# EUROPEAN PATENT APPLICATION

(11) **EP 4 696 358 A1**
(43) Date of publication of application: **18.02.2026**
(21) Application number: 24813931.3
(22) Date of filing: 16.04.2024
(51) Int. Cl.: A61M 25/08, A61B 17/00

(54) **BENDING CONTROL HANDLE AND INTERVENTIONAL SYSTEM**

(30) Priority: 26.05.2023 CN 202310610701
(71) Applicant: Shanghai MicroPort CardioFlow Medtech Co., Ltd., Shanghai 201203 (CN)
(72) Inventor: LU, Shigui, Shanghai 201203 (CN); LIN, Xing, Shanghai 201203 (CN); HUANG, Qingqing, Shanghai 201203 (CN); CHEN, Guoming, Shanghai 201203 (CN)
(74) Representative: Marks & Clerk LLP
(86) International application number: PCT/CN2024/087904
(87) International publication number: WO 2024/244725

(57) **Abstract**

The present invention provides a bending control handle and an interventional system. The bending control handle includes a driving part, a circumferential indication part, a transmission part and a bending control line connecting part. The bending control handle defines an axis. The driving part and the circumferential indication part are both configured so as to be rotatable circumferentially around the axis, and the bending control line connecting part is configured so as to be translatable along the axis. The transmission part is configured to convert the circumferential rotation of the driving part into both the translation of the bending control line connecting part along the axis and circumferential rotation of the circumferential indication part. An amount of the translation of the bending control line connecting part along the axis is in a predetermined ratio to an amount of the circumferential rotation of the circumferential indication part. With this arrangement, the circumferential indication part can provide an indication simply by its circumferential movement, thereby effecting space savings for the arrangement of other components of the bending control handle.

## Description

### TECHNICAL FIELD

The present invention relates to the field of medical devices and, in particular, to a bending control handle and an interventional system.

### BACKGROUND

Interventional therapy is a brand new therapeutic technique developed abroad in recent years, which allows for minimally invasive treatment based on modern high-tech advancements. It involves introducing a specialized, delicate instrument into the body of a patient under the guidance of medical imaging equipment to deliver diagnosis or local treatment to a target lesion site therein. This technique offers a variety of advantages including minor trauma, rapid recovery and good outcomes, and can minimize the patient trauma caused by traditional surgery.

Interventional systems typically consist of two components: a catheter and a handle. The catheter is configured for insertion into a patient's body, and the handle, serving as a power source for the entire interventional procedure and an actuation and control means, is required to ensure sufficient safety and efficiency. In order to facilitate delivery of interventional devices through tortuous blood vessels, there have been developed steerable catheters with a more bending control line being provided therein, which can be manipulated to bend and steer a tip portion of the catheter in any desired direction. The bending control line extending in the catheter is tied to a handle, which is configured for manipulation of the wire. However, since the bending control line is usually concealed within the handle, it is difficult to know its exact amounts of translational motion, potentially leading to inaccurate manipulation.

### SUMMARY

It is an object of the present invention to provide a bending control handle and an interventional system, which overcome the problem that it is difficult to know exact amounts of translational motion of a bending control line in conventional steerable catheters.

To solve the above technical problem, the present invention provides a bending control handle including a driving part, a circumferential indication part, a transmission part and a bending control line connecting part. The bending control handle defines an axis. The driving part and the circumferential indication part are both configured so as to be rotatable circumferentially around the axis, and the bending control line connecting part is configured so as to be translatable along the axis.

The transmission part is configured to convert the circumferential rotation of the driving part into both the translation of the bending control line connecting part along the axis and circumferential rotation of the circumferential indication part.

An amount of the translation of the bending control line connecting part along the axis is in a predetermined ratio to an amount of the circumferential rotation of the circumferential indication part.

Optionally, the transmission part may include a rotating shaft and an intermediary member, the rotating shaft coupled to the driving part and thereby rotatable circumferentially around the axis under the drive of the driving part, the rotating shaft engaging the intermediary member via first treads,
the intermediary member limited to making spiral motion around the axis, the intermediary member movably coupled to the circumferential indication part along the axis while being fixed in position circumferentially relative to the circumferential indication part,
wherein circumferential rotation of the rotating shaft causes the spiral motion of the intermediary member around the axis under the action of the first treads, which in turn causes the circumferential rotation of the circumferential indication part.

Optionally, the bending control handle may further include a first stop means, and the intermediary member may include a second stop means, wherein:
the first stop means extends spirally around the axis, with the second stop means being arranged so as to be movable along the extension of the first stop means relative thereto, or the second stop means extends spirally around the axis, with the first stop means being arranged so as to be movable along the extension of the second stop means relative thereto; and
the spiral motion of the intermediary member consists of its translation along the axis caused by the rotating shaft and its circumferential rotation caused by power transmission part and motion conversion of the first and second stop means.

Optionally, one of the first and second stop means may be a first recess, and the other may be a first protrusion movably engageable in the first recess, wherein the first protrusion and the first recess are matched in size along the axis so as to remain in abutment with each other.

Optionally, the intermediary member may include a third stop means, and the circumferential indication part may include a fourth stop means and be fixed in position along the axis, wherein:
the fourth stop means extends at an angle with respect to a direction of movement of the intermediary member, with the third stop means being configured so as to be movable relative to the fourth stop means along the extension thereof, or the third stop means extends at an angle with respect to the direction of movement of the intermediary member, with the fourth stop means being configured so as to be movable relative to the third stop means along the extension thereof; and
the circumferential rotation of the circumferential indication part is caused by power transmission part and motion conversion of the third and fourth stop means from the spiral motion of the intermediary member.

Optionally, one of the third and fourth stop means may be a second recess, and the other may be a second protrusion movably engaged in the second recess, wherein the second protrusion and the second recess are matched in size in a direction perpendicular to the extension of the fourth stop means so as to remain in abutment with each other.

Optionally, the rotating shaft may define a lumen extending therethrough along the axis, wherein: the intermediary member is disposed over the rotating shaft; the bending control line connecting part is arranged in the lumen; and the circumferential indication part is disposed over the intermediary member.

Optionally, the transmission part may include a rotating shaft, which is coupled to the driving part and thereby rotatable circumferentially under the drive of the driving part, wherein the rotating shaft engages the bending control line connecting part via second threads so that its circumferential rotation causes the translation of the bending control line connecting part along the axis under the action of the second threads.

Optionally, the transmission part may include a rotating shaft, which is coupled to the driving part and thereby rotatable circumferentially under the drive of the driving part, wherein the bending control handle includes a base and a plurality of balls arranged circumferentially around the axis, and the rotating shaft is coupled to the base along the axis via the plurality of balls.

Optionally, the bending control line connecting part may include a wire winding post and a wire pressing assembly, the wire winding post configured for winding of a bending control line thereon, the wire pressing assembly configured to retain the bending control line that has been wound on and then unwire winding posted from the wire winding post, wherein an axial direction of the wire winding post forms an angle with the axis, and the wire pressing assembly compressively retains the bending control line in a direction parallel to the axis.

Optionally, the bending control handle may include a housing provided therein with a circumferentially-extending observation window, wherein the circumferential indication part is disposed in the housing within the extent of the observation window.

Optionally, a transmission part ratio from the driving part to the circumferential indication part may be greater than 1.

To the above end, the present invention also provides an interventional system including the bending control handle as defined above and a bending control line, which is fastened to the bending control line connecting part and thereby translatable along the axis under the drive of the bending control line connecting part.

In summary, the present invention provides a bending control handle and an interventional system. The bending control handle includes a driving part, a circumferential indication part, a transmission part and a bending control line connecting part. The bending control handle defines an axis. The driving part and the circumferential indication part are both configured so as to be rotatable circumferentially around the axis, and the bending control line connecting part is configured so as to be translatable along the axis. The transmission part is configured to convert the circumferential rotation of the driving part into both the translation of the bending control line connecting part along the axis and circumferential rotation of the circumferential indication part. An amount of the translation of the bending control line connecting part along the axis is in a predetermined ratio to an amount of the circumferential rotation of the circumferential indication part.

With this arrangement, the driving part can be manipulated to cause translation of the bending control line connecting part along the axis and simultaneous circumferential rotation of the circumferential indication part, concurrently with the circumferential indication part providing an indication of an amount of translational motion of the bending control line connecting part. Moreover, since the circumferential indication part provides the indication simply by its circumferential movement, space savings are effected for the arrangement of other components of the bending control handle.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 is a schematic diagram of a bending control handle according to an embodiment of the present invention;
Fig. 2 is a schematic cross-sectional view of a bending control handle according to an embodiment of the present invention taken along an axis thereof;
Fig. 3 schematically illustrates a rotating shaft and a base according to an embodiment of the present invention;
Fig. 4 schematically illustrates first and second stop means according to an embodiment of the present invention;
Fig. 5 schematically illustrates amounts of translational motion of a bending control line connecting part and an intermediary member according to an embodiment of the present invention;
Fig. 6 schematically illustrates a direction of movement of an intermediary member according to an embodiment of the present invention;
Fig. 7 is a schematic diagram of a circumferential indication part according to an embodiment of the present invention;
Fig. 8 schematically illustrates a direction of movement of a circumferential indication part according to an embodiment of the present invention; and
Fig. 9 is a schematic diagram of a bending control line connecting part according to an embodiment of the present invention.

### List of Reference Numerals

1 driving part; 11 knob; 2 circumferential indication part; 24 fourth stop means; 3 transmission part; 31 rotating shaft; 311 first external thread; 32 intermediary member; 322 second stop means; 323 third stop means; 4 bending control line connecting part; 41 second external thread; 42 wire winding post; 43 wire pressing assembly; 44 protective sleeve; 45 threaded section; 46 follower section; 50 base; 51 housing; 52 observation window; 53 first stop means; 6 bending control line; 71 catheter; 72 catheter connecting part; 8 ball.

### DETAILED DESCRIPTION

Objects, advantages and features of the present invention will become more apparent upon reading the following more detailed description of specific embodiments thereof in conjunction with the accompanying drawings. Note that the figures are provided in a very simplified form not necessarily drawn to exact scale for the only purpose of helping explain embodiments of the invention disclosed herein in a more convenient and clearer way. In addition, the illustrated structures are usually part of their real-world counterparts. In particular, as the figures tend to have distinct emphases, they are sometimes drawn to different scales.

As used herein, the singular forms "a", "an" and "the" include plural referents, and the term "or" is generally employed in the sense of "and/or", "a number of" of "at least one" and "at least two" of "two or more". Additionally, the use of the terms "first", "second" and "third" herein is intended to be illustrative only and is not to be construed as denoting or implying relative importance or as implicitly indicating the number of the referenced items. Accordingly, defining an item with "first", "second" or "third" is an explicit or implicit indication of the presence of one or at least two such items. The terms "one end" and "other end", as well as "proximal end" and "distal end", may be used herein to generally refer to opposite end portions including the endpoints, rather than only to the endpoints. As used herein, the terms "proximal end" and "distal end" may be used with respect to an interventional system, with one end being inserted inside a patient's body (e.g., provided by a steerable catheter) and another end extending outside the body for manipulation (e.g., provided by a bending control handle). In this regard, when used in relation to a component, the term "proximal end" may refer to a location thereon closer to the manipulation end of the interventional system that extends outside the patient's body, while the term "distal end" may refer to a location on the component, which is closer to the end of the interventional system that is inserted within the patient's body and therefore farther away from the manipulation end of the interventional system. The terms "proximal end" and "distal end" may also be used to describe a manual operation, or an operation conducted by hand. In this sense, the "proximal end" and "distal end" may be used herein with respect to an operator, such as a surgeon or clinician. Specifically, when used in relation to a component, the term "proximal end" may refer to a location thereon closer to the operator, while the term "distal end" may refer to a location on the component, which is closer to the interventional system and therefore farther away from the operator. Further, as used herein, the terms "mounting", "coupling", "connecting", "disposing" and any variants thereof should be interpreted in a broad sense. When an element is referred to as being "disposed" on another element, this is generally intended to only mean that there is a connection, coupling, engagement or transmission part relationship between the two elements, which may be either direct or indirect with one or more intervening elements, and should not be interpreted as indicating or implying a particular spatial position relationship between them. That is, the element may be located inside, outside, above, under, beside, or at any other location relative to the other element, unless the context clearly dictates otherwise. Those of ordinary skill in the art can understand the specific meanings of the above-mentioned terms herein, depending on their context. Furthermore, the directional terms such as "above", "below", "upper", "lower", "upward", "downward", "left", "right", and the like are used in relation to the illustrative embodiments as they are depicted in the figures, the upward or upper direction being toward the top of the corresponding figure and the downward or lower direction being toward the bottom of the corresponding figure.

It is an object of the present invention to provide a bending control handle and an interventional system, which overcome the problem that it is difficult to know exact amounts of translational motion of a bending control line in conventional steerable catheters.

As described in the Background section, conventionally, it is difficult to know exact amounts of translational motion of a bending control line. Considering such a bending control line is usually translated along an axis of a bending control handle, one skilled in the art might add something to the bending control handle that allows observing or indicates exact amounts of translational motion of the bending control line. However, this would occupy a considerable space of the bending control handle, affecting the layout of its other components.

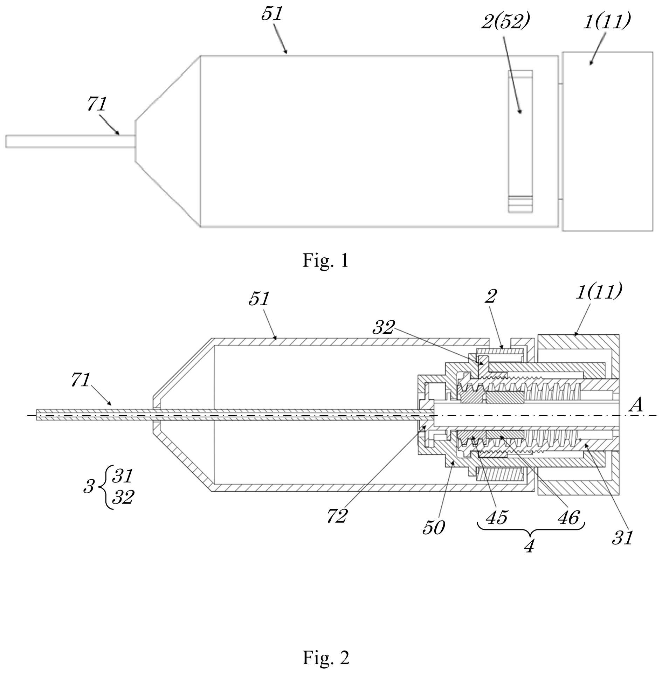

In view of this, referring to Figs. 1 to 9, embodiments described herein provide a bending control handle including a driving part 1, a circumferential indication part 2, a transmission part 3 and a bending control line connecting part 4. The bending control handle defines an axis A. Both the driving part 1 and the circumferential indication part 2 are arranged so as to be rotatable circumferentially around the axis A, and the bending control line connecting part 4 is disposed so as to be translatable along the axis A. The transmission part 3 can convert circumferential rotation of the driving part 1 into both translation of the bending control line connecting part 4 along the axis A and circumferential rotation of the circumferential indication part 2. An amount of translational motion of the bending control line connecting part 4 along the axis A is in a predetermined ratio to an amount of circumferential rotation of the circumferential indication part 2. This predetermined ratio may be selected as desired. Preferably, the predetermined ratio is linearly adjustable.

With this arrangement, the driving part 1 may be manipulated to translate the bending control line connecting part 4 along the axis A, thereby causing simultaneous circumferential rotation of the circumferential indication part 2, concurrently with the circumferential indication part 2 providing an indication of an amount of translational motion of the bending control line connecting part 4. Since the circumferential indication part 2 provides the indication simply by its circumferential movement, space savings are effected for the arrangement of other components of the bending control handle.

The proposed bending control handle will be described below by way of example with reference to the accompanying drawings. For ease of description, the term "axial" may be used hereinafter to refer to a direction along the axis A.

Referring to Figs. 1, 2 and 4, in an optional embodiment, the bending control handle includes a housing 51 and a base 50. The base 50 is arranged along the axis so as to be partially received in the housing 51 and partially extend outside of the housing 51. The base 50 defines an axially extending lumen, and the bending control line connecting part 4 is provided within the lumen. Part of the transmission part 3 is received in the lumen of the base 50, while the rest remains out of the base 50. The circumferential indication part 2 is disposed on the exterior of the base 50.

Additionally, the housing 51 is provided therein with a circumferentially-extending observation window 52, and the circumferential indication part 2 is disposed in the housing 51 within the extent of the observation window 52. Thus, as would be appreciated, circumferential rotation of the circumferential indication part 2 could be visually observed through the observation window 52. Optionally, the observation window 52 may be a cut-through slot, optionally covered with a piece of transparent material. Alternatively, the housing 51 may be even made of a transparent material in entirety or in part. In this case, the observation window 52 may be integral with the rest of the housing 51, or part thereof. Preferably, the circumferential indication part 2 includes a marker, such as a pointer or graduated scale, and the housing 51 is provided with circumferentially spaced graduation lines, which serve along with the marker to provide an indication of an amount of circumferential rotation of the circumferential indication part 2.

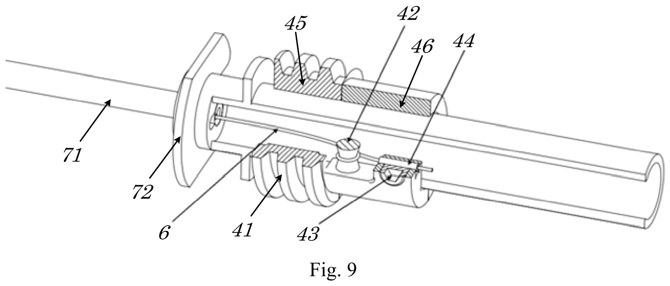

With combined reference to Figs. 2 and 9, in one exemplary embodiment, the bending control handle is further coupled to a catheter 71, the catheter 71 may be implemented as a conventional steerable catheter, without departing from the scope of the present invention. The catheter 71 may be coupled to the base 50, for example, by a catheter connecting part 72. The bending control line connecting part 4 is for connecting the proximal end of the bending control line 6. The distal end of the bending control line 6 passes into the catheter 71 and extends distally. With this arrangement, axially moving the bending control line connecting part 4 can cause axial translational motion of the bending control line 6, thereby achieving bending and steering of the catheter 71.

Optionally, referring to Figs. 2 to 6, the transmission part 3 may include a rotating shaft 31 and an intermediary member 32. The rotating shaft 31 is coupled to the driving part 1. The rotating shaft 31 is rotatable circumferentially around the axis A under the drive of the driving part 1. The rotating shaft 31 and the intermediary member 32 engage each other via first treads, movement of the intermediary member 32 is limited to spiral motion around the axis A. The intermediary member 32 is movably coupled to the circumferential indication part 2 along the axis A. The intermediary member 32 is fixed in position circumferentially relative to the circumferential indication part 2. Circumferential rotation of the rotating shaft 31 causes spiral motion of the intermediary member 32 around the axis A under the action of the first treads, the spiral motion of the intermediary member 32 causes circumferential rotation of the circumferential indication part 2.

In one optional embodiment, the driving part 1 includes a knob 11, the knob 11 is coaxially coupled to the rotating shaft 31, allowing the rotating shaft 31 to be circumferentially rotated about the axis A by turning the knob 11. The intermediary member 32 is provided as a cylinder member disposed on the exterior of the rotating shaft 31. The first treads include a first external thread 311 on an outer circumference of the rotating shaft 31 and a first internal thread in an inner circumference of the intermediary member 32. The engagement of the rotating shaft 31 and the intermediary member 32 via the first treads can be accomplished by intermeshing of the first external thread 311 and the first internal thread.

As noted above, movement of the intermediary member 32 is limited to spiral motion around the axis A, which is converted by the first treads from rotation of the rotating shaft 31. Such spiral motion of the intermediary member 32 around the axis A consists of an axial component and a circumferential component. Since the intermediary member 32 is movably coupled to the circumferential indication part 2 along the axis, the axial component of the spiral motion of the intermediary member 32 is cancelled out by the axial motion of the intermediary member 32 relative to the circumferential indication part 2. Meanwhile, since the intermediary member 32 is fixed in position circumferentially relative to the circumferential indication part 2, the circumferential component of the spiral motion of the intermediary member 32 causes the circumferential indication part 2 to circumferentially rotate to provide an indication.

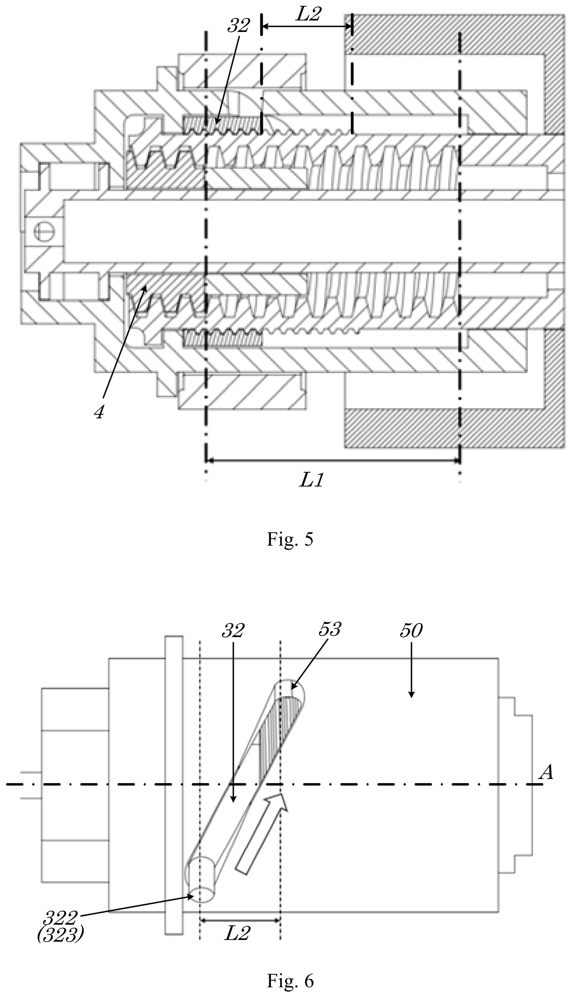

Optionally, the bending control handle may include a first stop means 53, and the intermediary member 32 may include a second stop means 322. The first stop means 53 may helically extend around the axis A, and the second stop means 322 may be arranged so as to be movable along the extension of the first stop means 53 relative to the first stop means 53. Alternatively, the second stop means 322 may helically extend around the axis A, and the first stop means 53 may be arranged so as to be movable along the extension of the second stop means 322 relative to the second stop means 322. When the intermediary member 32 is driven by the rotating shaft 31 to move along the axis A, the first stop means 53 and the second stop means 322 interact to provide power transmission part and motion conversion for causing simultaneous circumferential rotation of the intermediary member 32, leading to spiral motion as indicated by the arrow of Fig. 6.

The first stop means 53 and the second stop means 322 are cooperatively paired to work together to limit the intermediary member 32 to spiral motion around the axis A. Preferably, the first stop means 53 is fixed in the housing 51 or integrally formed with the housing 51. In some embodiments, the first stop means 53 may be shaped like a track way helically extending around the axis A. In these embodiments, the movability of the second stop means 322 along the extension of the first stop means 53 is intended to mean that the second stop means 322 can move only along the extension of the first stop means 53 without deviating from the extent of the latter, like a vehicle running on a track way. In this way, movement of the intermediary member 32 can be limited as described above.

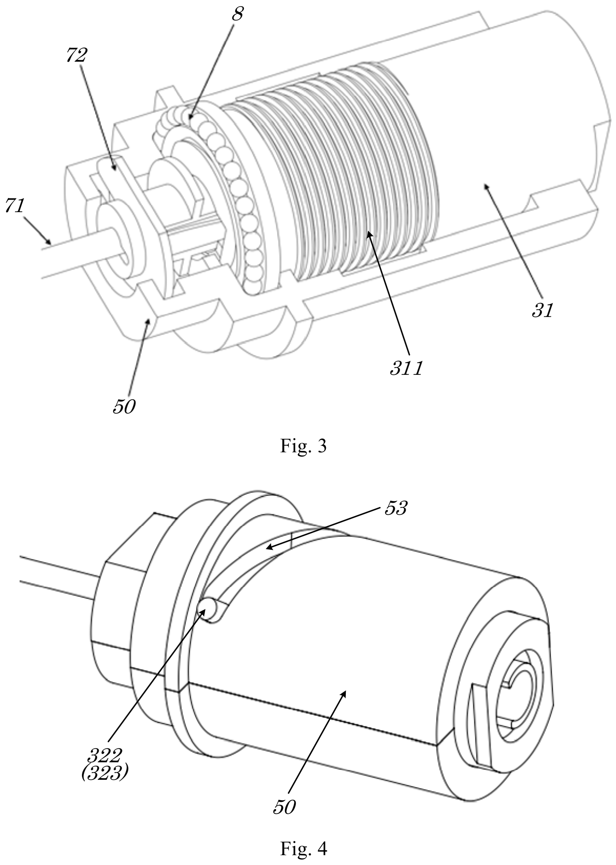

Optionally, one of the first stop means 53 and the second stop means 322 may be a first recess, and the other may be a first protrusion movably engageable in the first recess. Along the axis A, the first protrusion and the first recess may be matched in size so as to remain in abutment with each other. Referring to Fig. 4, in one optional embodiment, the intermediary member 32 is movably disposed within the lumen of the base 50. Moreover, the first stop means 53 is a helical slot formed in the base 50, and the first protrusion is movably received in the first recess. Further, an axial dimension of the first protrusion matches an axial dimension of the first recess (which may be considered as a width of the first recess) so that the first protrusion remains in contact with axially opposing side walls of the first recess. With this arrangement, movement of the first protrusion is limited to motion along the extension of the first recess, and movement of the intermediary member 32 is hence limited to spiral motion within the first recess.

In an alternative embodiment, the first stop means 53 is the first protrusion, and the second stop means 322 is the first recess. In particular, the first stop means 53, i.e., the first protrusion, may helically extend and, for example, be raised inwardly into the lumen of the base 50. In addition, an axial width of the first recess matches an axial width of the first protrusion so that the first protrusion can be snuggly received in the first recess and slide therein. With this arrangement, movement of the intermediary member 32 may also be limited to spiral motion as discussed above.

In all the foregoing embodiments, the first stop means 53 extends helically around the axis A, and the second stop means 322 is provided in the first stop means 53 or on the first stop means 53 so as to be movable along the extension of the first stop means 53. It is understood that, in some other embodiments, it is also possible that the second stop means 322 of the intermediary member 32 extends helically around the axis A, with the first stop means 53 being provided in the second stop means 322 or on the second stop means 322 so as to be movable along the extension of the second stop means 322. For example, the second stop means 322 may be implemented as the first recess and extend helically, and the first stop means 53 as the first protrusion. With this arrangement, similar effects can be achieved.

It should be noted that the first stop means 53 and the second stop means 322 are not limited to being implemented as a protrusion/recess pair. In some alternative embodiments, the first stop means 53 and the second stop means 322 may be structures capable of providing the limitation function by mutual contact and abutment, such as a pair of complementary steps. Yet alternatively, they may be structures capable of providing the limitation function contactlessly by magnetic attraction, such as magnets. For example, the first stop means 53 may be a helically extending iron bar, and the second stop means 322 may be a magnet arranged in opposition to the first stop means 53. According to such embodiments, movement of the intermediary member 32 can also be limited as described above. Those skilled in the art will understand the foregoing and other implementations that enable the above-discussed function based on the common general knowledge in the art, and further description in this regard is omitted herein.

Referring to Figs. 4 to 8, optionally, the intermediary member 32 may include a third stop means 323, and the circumferential indication part 2 may include a fourth stop means 24. The circumferential indication part 2 is fixed in position along the axis A. The fourth stop means 24 may extend at an angle with respect to movement of the intermediary member 32, and the third stop means 323 may be configured so as to be movable relative to the fourth stop means 24 along the extension thereof. Alternatively, the third stop means 323 may extend at an angle with respect to movement of the intermediary member 32, and the fourth stop means 24 may be configured so as to be movable relative to the third stop means 323 along the extension thereof. When the intermediary member 32 makes spiral motion, the third stop means 323 and the fourth stop means 24 interact to provide power transmission part and motion conversion for causing circumferential rotation of the circumferential indication part 2.

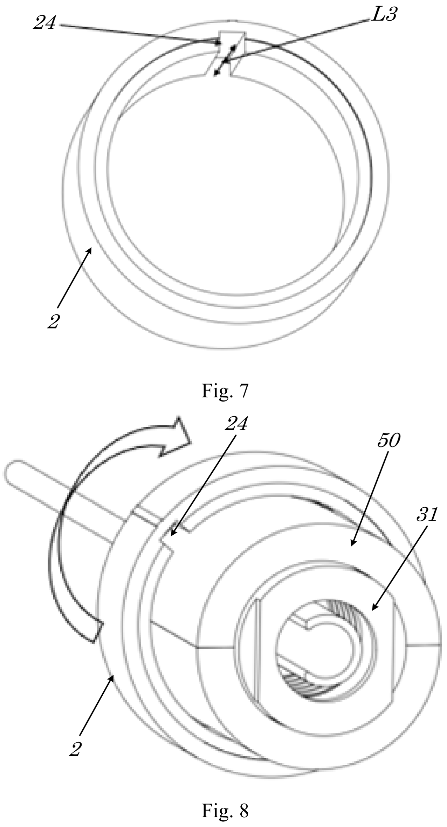

The third stop means 323 and the fourth stop means 24 are cooperatively paired to work together to enable circumferential rotation of the circumferential indication part 2 as a result of spiral motion of the intermediary member 32 while cancelling out an axial component of the spiral motion of the intermediary member 32. Referring to Figs. 7 and 8, in one optional embodiment, the circumferential indication part 2 is cylindrical and axially disposed on the exterior of the base 50 and hence over the intermediary member 32. That is, the circumferential indication part 2 is disposed over the intermediary member 32, with the base 50 being situated therebetween. Of course, in some other embodiments, the base 50 may cover only part of the intermediary member 32, with the circumferential indication part 2 being instead directly disposed over the exterior of the rest of the intermediary member 32. That is to say, it is also possible for the circumferential indication part 2 to be directly disposed on the exterior of the intermediary member 32.

In some embodiments, the fourth stop means 24 extends at an angle with respect to movement of the intermediary member 32, and the third stop means 323 makes spiral motion along with the intermediary member 32. Therefore, it moves at an angle with respect to the extension of the fourth stop means 24. The spiral motion of the third stop means 323 consists of a first component parallel to the extension of the fourth stop means 24 and a second component perpendicular to the extension of the fourth stop means 24. Since the third stop means 323 is movable along the extension of the fourth stop means 24, the first component is cancelled out, without affecting the motion of intermediary member 32 relative to the circumferential indication part 2. Meanwhile, the second component drives the circumferential indication part 2 to move. Moreover, since the circumferential indication part 2 is fixed in position along the axis, the circumferential indication part 2 can only rotate circumferentially. It is understood that the circumferential indication part 2 can be urged by the third stop means 323 to rotate circumferentially as long as the fourth stop means 24 extends at an angle with respect to movement of the intermediary member 32. The smaller the angle at which the fourth stop means 24 extends with respect to movement of the intermediary member 32 is, the smaller an amount of circumferential rotation of the circumferential indication part 2 will be caused by the movement of the intermediary member 32. When the fourth stop means 24 extends at an angle of 0° with respect to movement of the intermediary member 32, the circumferential indication part 2 will not circumferentially rotate at all even when the intermediary member 32 moves. That is, the intermediary member 32 will rotate idly without causing movement of the circumferential indication part 2. On the contrary, the larger the angle at which the fourth stop means 24 extends with respect to movement of the intermediary member 32 is, the greater an amount of circumferential rotation of the circumferential indication part 2 will be caused by the movement of the intermediary member 32. When the fourth stop means 24 extends at an angle of 90° with respect to movement of the intermediary member 32, the intermediary member 32 will be stuck onto the circumferential indication part 2 and prevented from any movement. It will be appreciated from the above analysis that the angle at which the fourth stop means 24 extends with respect to movement of the intermediary member 32 may be selected between 0° and 90°, but not as 0° or 90°. Those skilled in the art can appropriately select the angle at which the fourth stop means 24 extends with respect to movement of the intermediary member 32, as required.

Optionally, one of the third stop means 323 and the fourth stop means 24 may be a second recess, and the other may be a second protrusion movably engaged in the second recess. The second protrusion and the second recess may be matched in size perpendicular to the extension of the fourth stop means 24 so as to remain in abutment with each other.

Referring to Figs. 4 and 6 to 8, in one optional embodiment, the third stop means 323 is the second protrusion, and the fourth stop means 24 is the second recess. The second recess extends parallel to the axis A, and the second protrusion is movably received in the second recess. Further, a dimension of the second protrusion measured perpendicular to extension of the fourth stop means 24 matches a dimension of the second recess measured in the same direction (which may be considered as a circumferential width of the second recess) so that the second protrusion remains in circumferentially opposing side walls of the second recess. With this arrangement, movement of the second protrusion is limited to spiral motion in the second recess along the extension thereof (i.e., axial motion) along with the intermediary member 32, a circumferential component of which causes circumferential rotation of the circumferential indication part 2. It is understood that an axial length L3 of the second recess should accommodate a maximum possible amount of translational motion L2 of the second protrusion in the same direction caused by spiral motion of the intermediary member 32, in order to prevent dislodgement of the second protrusion from the second recess.

It should be noted that, in some embodiments, the third stop means 323 and the second stop means 322 may be the same stop means. For example, in the exemplary embodiment of Figs. 4 and 6 to 8, the second stop means 322, i.e., the first protrusion, and the third stop means 323, i.e., the second protrusion, may be the same protrusion serving as both the second stop means 322 and the third stop means 323. Of course, in alternative embodiments, the third stop means 323 and the second stop means 322 may be separate structures.

In an alternative embodiment, the fourth stop means 24 is the second protrusion, and the third stop means 323 is the second recess. Specifically, in this case, the fourth stop means 24, i.e., the second protrusion, may extend along the axis, and the second protrusion may be, for example, raised inwardly from the inner circumference of the circumferential indication part 2. The second protrusion may be slidably received in the second recess, thereby enabling circumferential rotation of the circumferential indication part 2 due to spiral motion of the intermediary member 32 in a similar way to the exemplary embodiment of Figs. 4 and 6 to 8. Therefore, further description in this regard is omitted herein.

In all the foregoing embodiments, the fourth stop means 24 extends at an angle with respect to movement of the intermediary member 32, and the third stop means 323 is configured so as to be movable relative to the fourth stop means 24 along the extension of the fourth stop means 24. It is understood that, in some other embodiments, it is also possible that the third stop means 323 extends at an angle with respect to movement of the intermediary member 32, with the fourth stop means 24 being configured so as to be movable relative to the fourth stop means 24 along the extension thereof. For example, the third stop means 323 may be implemented as the second recess and extend axially, and the fourth stop means 24 as the second protrusion. With this arrangement, similar effects can be achieved.

It should be noted that the third stop means 323 and the fourth stop means 24 are not limited to being implemented as a protrusion/recess pair. In some alternative embodiments, the third stop means 323 and the fourth stop means 24 may be structures capable of providing the limitation function by mutual contact and abutment, such as a pair of complementary steps. Yet alternatively, they may be structures capable of providing the limitation function contactlessly by magnetic attraction, such as magnets. Those skilled in the art will understand the foregoing and other implementations that enable the above-discussed function based on the common general knowledge in the art, and further description in this regard is omitted herein.

Referring to Fig. 9, in conjunction with Fig. 2, the rotating shaft 31 may optionally engage the bending control line connecting part 4 via second threads so that the bending control line connecting part 4 is urged to move along the axis A under the action of the second threads as a result of circumferential rotation of the rotating shaft 31. In one optional embodiment, the rotating shaft 31 defines a lumen extending therethrough along the axis A, and the bending control line connecting part 4 is disposed within the lumen.

Optionally, the second threads may include a second internal thread provided in the lumen of the rotating shaft 31 and a second external thread 41 on an outer circumference of the bending control line connecting part 4. The engagement of the rotating shaft 31 and the bending control line connecting part 4 via the first treads can be accomplished by intermeshing of the second external thread 41 and the second internal thread.

Further, movement of the bending control line connecting part 4 is limited to translational motion along the axis A. In other words, rotation of the rotating shaft 31 can be converted by the second threads into axial translation of the bending control line connecting part 4 and hence of the bending control line 6 fastened to the bending control line connecting part 4, thus achieving bending and steering.

Preferably, referring to Fig. 5, an axial length ratio of the second internal thread to the first external thread 311 is equal to a pitch ratio of the second internal thread to the first external thread 311. This enables the bending control line connecting part 4 to axially translate a distance L1 compatible with a distance L2 the intermediary member 32 axially translates, thus preventing, for example, the intermediary member 32 from reaching a limit of its axial translation before the bending control line connecting part 4 completes its axial travel. When this happens, the intermediary member 32 may get stuck.

Optionally, the bending control line connecting part 4 may include a wire winding post 42 and a wire pressing assembly 43. The wire winding post 42 is configured for winding of the bending control line 6 thereon, and the wire pressing assembly 43 is configured to retain the bending control line 6 that has been wound on and then unwire winding posted from the wire winding post 42. The wire winding post 42 is disposed with its axis forming an angle with the axis A, and the wire pressing assembly 43 compressively retains the wire in a direction parallel to the axis A. After being proximally passed out of the catheter 71, the bending control line 6 is wound on the wire winding post 42, unwire winding posted therefrom and compressively retained by the wire pressing assembly 43. With this arrangement, buffered by the wire winding post 42, stress on the bending control line 6 can be reduced, mitigating stress concentration at a portion of the bending control line 6, where it is retained by the wire pressing assembly 43. Preferably, the bending control line connecting part 4 further includes a protective sleeve 44, the protective sleeve 44 is disposed over a portion of the bending control line 6 aligned with the wire pressing assembly 43 to provide protection to the bending control line 6, thereby reducing or preventing damage to the bending control line 6 during its compressive retention by the wire pressing assembly 43.

Optionally, in some embodiments, the bending control line connecting part 4 may include a threaded section 45 and a follower section 46. The second external thread 41 may be defined on the threaded section 45, and the wire winding post 42, the wire pressing assembly 43 and the protective sleeve 44 may be all provided on the follower section 46. Without limitation, the threaded section 45 and the follower section 46 may be, for example, simply brought into abutment with each other, or fixedly coupled to each other, along the axis.

Optionally, referring to Figs. 2 and 3, the bending control handle may include multiple balls 8 arranged circumferentially around the axis A, and the rotating shaft 31 may be coupled to the base 50 via the balls 8 along the axis A. The rotating shaft 31 is axially fixed in position, and when driven by the driving part 1 to rotate circumferentially, urged by the bending control line connecting part 4 and the intermediary member 32. Therefore, it may be subject to axial stress from the base 50. The presence of the balls 8 allows for rolling friction between rotating shaft 31 and the base 50, which can greatly improve transmission part efficiency and result in reductions in loss during bending and steering, operating torques and noise.

Preferably, a transmission part ratio from the driving part 1 to the circumferential indication part 2 is greater than 1. Power transmission part from the driving part 1 to the circumferential indication part 2 may be considered as speed-reducing transmission part. Thus, in response to the knob 11 being turned by an angle, the circumferential indication part 2 is rotated by an angle smaller than the angle. This allows for a smaller amount of rotation of the circumferential indication part 2 and a smaller area of the observation window 52. Thus, the indication functionality is allowed to take up a reduced space, enabling the bending control handle to accommodate additional functions.

It is understood that the transmission part ratio from the driving part 1 to the circumferential indication part 2 and the predetermined ratio of axial translation of the bending control line connecting part 4 to circumferential rotation of the circumferential indication part 2 may depend on multiple factors, particularly including a pitch ratio of the second threads to the first treads, the direction of movement of the intermediary member 32 and the angle at which the fourth stop means 24 extends with respect to the direction of movement of the intermediary member 32. That is, the transmission part ratio from the driving part 1 to the circumferential indication part 2 and the predetermined ratio may be modified by adjusting any of those factors.

On the basis of the bending control handle as discussed above, embodiments of the present invention further provide an interventional system incorporating the bending control handle. The interventional system further includes a bending control line 6, the bending control line 6 is fastened to the bending control line connecting part 4 and thereby translatable along the axis A under the drive of the bending control line connecting part 4. Optionally, the interventional system may further include a catheter 71, a guide wire and other necessary components as known in the art, and further description thereof is omitted herein. The proposed interventional system may be used in a variety of applications, including interventional treatment of heart valve diseases, as a non-limiting example.

In summary, the present invention provides a bending control handle and an interventional system. The bending control handle includes a driving part, a circumferential indication part, a transmission part and a bending control line connecting part. The bending control handle defines an axis. The driving part and the circumferential indication part are both configured so as to be rotatable circumferentially around the axis, and the bending control line connecting part is configured so as to be translatable along the axis. The transmission part is configured to convert the circumferential rotation of the driving part into both the translation of the bending control line connecting part along the axis and circumferential rotation of the circumferential indication part. An amount of the translation of the bending control line connecting part along the axis is in a predetermined ratio to an amount of the circumferential rotation of the circumferential indication part. With this arrangement, the driving part can be manipulated to cause translation of the bending control line connecting part along the axis and simultaneous circumferential rotation of the circumferential indication part, concurrently with the circumferential indication part providing an indication of an amount of translational motion of the bending control line connecting part. Moreover, since the circumferential indication part provides the indication simply by its circumferential movement, space savings are effected for the arrangement of other components of the bending control handle.

It should be noted that the foregoing embodiments may be combined in any suitable combination. The description presented above is merely that of a few preferred embodiments of the present invention and is not intended to limit the scope thereof in any sense. Any and all changes and modifications made by those of ordinary skill in the art based on the above teachings fall within the scope of the invention.

## Claims

1. A bending control handle, comprising a driving part, a circumferential indication part, a transmission part and a bending control line connecting part, the bending control handle defining an axis, the driving part and the circumferential indication part both configured to be rotatable around the axis in a circumferential direction, the bending control line connecting part configured to be movable along the axis,
the transmission part configured to convert the circumferential rotation of the driving part into the movement of the bending control line connecting part along the axis, and the transmission part further configured to convert the circumferential rotation of the driving part into circumferential rotation of the circumferential indication part,
wherein an amount of the movement of the bending control line connecting part along the axis is in a predetermined ratio to an amount of the circumferential rotation of the circumferential indication part.

2. The bending control handle according to claim 1, wherein the transmission part comprises a rotating shaft and an intermediary member, the rotating shaft connected to the driving part and thereby rotatable around the axis in the circumferential direction under the drive of the driving part, the rotating shaft engaging the intermediary member via first treads,
a movement direction of the intermediary member being limited to extend spirally around the axis, the intermediary member movably connected to the circumferential indication part along the axis, and the position of the intermediary member relative to the circumferential indication part in the circumferential direction is constrained,
wherein circumferential rotation of the rotating shaft causes the intermediary member to move spirally around the axis under the action of the first treads, and the spiral movement of the intermediary member causes the circumferential rotation of the circumferential indication part.

3. The bending control handle according to claim 2, further comprising a first stop means, the intermediary member comprising a second stop means, wherein:
the first stop means extends spirally around the axis, the second stop means is arranged to be movable relative to the first stop means along an extension direction of the first stop means, or the second stop means extends spirally around the axis, the first stop means is arranged to be movable relative to the second stop means along an extension direction of the second stop means; and
when the intermediate member moves along the axis under the drive of the rotating shaft, based on the transmission conversion between the first stop means and the second stop means, it synchronously generates circumferential rotation, forming a spiral motion.

4. The bending control handle according to claim 3, wherein one of the first stop means and the second stop means is a first recess, with the other being a first protrusion, the first protrusion movably engageable in the first recess, wherein the first protrusion and the first recess are matched in size along the axis and abut against each other.

5. The bending control handle according to claim 2, wherein: the intermediary member comprises a third stop means, the circumferential indication part comprises a fourth stop means; the position of the circumferential indication part along the axis is constrained,
the fourth stop means extends at an angle with respect to a direction of movement of the intermediary member, the third stop means is configured to be movable relative to the fourth stop means along an extension direction of the fourth stop means, or the third stop means extends at an angle with respect to the direction of movement of the intermediary member, the fourth stop means is configured to be movable relative to the third stop means along an extension direction of the third stop means; and
when the intermediary member moves spirally, based on transmission conversion between the third stop means and the fourth stop means, it drives the circumferential indication part to rotate circumferentially.

6. The bending control handle according to claim 5, wherein one of the third stop means and the fourth stop means is a second recess, with the other being a second protrusion movably engaged in the second recess, wherein the second protrusion and the second recess are matched in size in a direction perpendicular to the extension direction of the fourth stop means and abut against each other.

7. The bending control handle according to claim 2, wherein: the rotating shaft defines a lumen extending therethrough along the axis, the intermediary member is disposed over the rotating shaft; the bending control line connecting part is arranged in the lumen; and the circumferential indication part is disposed over the intermediary member.

8. The bending control handle according to claim 1, wherein the transmission part comprises a rotating shaft, the rotating shaft is connected to the driving part, the rotating shaft rotatable in the circumferential direction under the drive of the driving part, wherein the rotating shaft engages the bending control line connecting part via second threads; when the rotating shaft rotates circumferentially, it drives the bending control line connecting part to move along the axis under the action of the second threads.

9. The bending control handle according to claim 1, wherein the transmission part comprises a rotating shaft; the rotating shaft is connected to the driving part, the rotating shaft rotatable in the circumferential direction under the drive of the driving part, wherein the bending control handle comprises a base and a plurality of balls arranged circumferentially around the axis, and the rotating shaft is connected to the base along the axis via the plurality of balls.

10. The bending control handle according to claim 1, wherein the bending control line connecting part comprises a wire winding post and a wire pressing assembly, the wire winding post configured for winding a bending control line around, the wire pressing assembly configured to retain the bending control line after it passes around the wire winding post, wherein an axial direction of the wire winding post forms an angle with the axis, and the wire pressing assembly compressively retains the bending control line in a direction parallel to the axis.

11. The bending control handle according to claim 1, wherein the bending control handle comprises a housing, the housing provided therein with a circumferentially-extending observation window, wherein the circumferential indication part is disposed in the housing and within the extent of the observation window.

12. The bending control handle according to claim 1, wherein a transmission part ratio from the driving part to the circumferential indication part is greater than 1.

13. An interventional system comprising the bending control handle according to any one of claims 1 to 12; the interventional system further comprising a bending control line, wherein the bending control line is connected to the bending control line connecting part and thereby translatable along the axis under the drive of the bending control line connecting part.
